# FASCICULE DE BREVET EUROPEEN

(11) **EP 3 716 989 B1**
(45) Date de publication et mention de la délivrance du brevet: **29.05.2024**
(21) Numéro de dépôt: 18811826.9
(22) Date de dépôt: 03.12.2018
(51) Int. Cl.: A61K 36/28, A61P 17/04, A61P 17/10, A61P 17/06

(54) **EXTRAIT D'HELICHRYSUM GYMNOCEPHALUM POUR LE TRAITEMENT ET/OU LA PRÉVENTION DES DERMATOSES INFLAMMATOIRES**
EXTRAKT AUS HELICHRYSUM GYMNOCEPHALUM ZUR BEHANDLUNG UND/ODER VORBEUGUNG VON ENTZÜNDLICHEN DERMATOSEN
EXTRACT OF HELICHRYSUM GYMNOCEPHALUM FOR THE TREATMENT AND/OR PREVENTION OF INFLAMMATORY DERMATOSES

(30) Priorité: 01.12.2017 FR 1761515
(43) Date de publication de la demande: 07.10.2020
(73) Titulaire: Pierre Fabre Dermo-Cosmétique, 81500 Lavaur (FR)
(72) Inventeur: MANDEAU, Anne, 31200 Toulouse (FR); HERNANDEZ-PIGEON, Hélène, 31270 Cugnaux (FR); ARIES, Marie Françoise, 31750 Escalquens (FR)
(74) Mandataire: Regimbeau
(86) Numéro de dépôt international: PCT/EP2018/083294
(87) Numéro de publication internationale: WO 2019/106203

(56) Documents cités:
- WO-A1-2012/098134
- WO-A1-2015/049666
- WO-A1-2016/120241
- JP-A- 2009 096 761
- CHOI JIN KYEONG ET AL: "Inhibitory effect of galangin on atopic dermatitis-like skin lesions", FOOD AND CHEMICAL TOXICOLOGY, vol. 68, 25 mars 2014 (2014-03-25), pages 135-141, XP028664179, ISSN: 0278-6915, DOI: 10.1016/J.FCT.2014.03.021
- CAVALLI J-F ET AL: "Constituents of the essential oil of six Helichrysum species from Madagascar", FLAVOUR AND FRAGRANCE JOURNAL, WILEY, NEW YORK, NY, GB, vol. 16, no. 4, 1 juillet 2001 (2001-07-01), pages 253-256, XP002655247, ISSN: 0882-5734, DOI: 10.1002/FFJ.994 [extrait le 2001-05-08]
- AFOULOUS S ET AL: "Helichrysum gymnocephalum essential oil: chemical composition and cytotoxic, antimalarial and antioxidant activities, attribution of the activity origin by correlations", MOLECULES: A JOURNAL OF SYNTHETIC ORGANIC AND NATURAL PRODUCT CHEMISTRY, M D P I AG, CH, vol. 16, no. 10, 29 septembre 2011 (2011-09-29), pages 8273-8291, XP002719363, ISSN: 1420-3049, DOI: 10.3390/MOLECULES16108273

## Description

La présente invention concerne un extrait d'Helichrysum gymnocephalum, ainsi qu'une composition dermatologique ou dermo-cosmétique, pour leur utilisation dans le traitement et/ou la prévention de dermatoses inflammatoires.

Le vaste genre Helichrysum contient plus de 600 espèces de plantes vivaces ou annuelles, herbacées, buissonnantes ou arbustives aux feuillages souvent aromatiques. Ce genre fait partie de la famille des Astéracées. Les Helichrysum sont très présents en Europe du sud, mais aussi en Asie, en Afrique du nord et australe, à Madagascar et en Australie. Il comprend quelques espèces orophiles caractéristiques des hauts massifs d'Afrique tropicale. A Madagascar, 115 espèces, toutes endémiques de la grande île, sont actuellement répertoriées. La plupart des autres espèces se trouvent dans les pays du pourtour méditerranéen, l'Europe occidentale, la Nouvelle Zélande.

Helichrysum gymnocephalum (DC) Humbert ou immortelle à tête nue a pour synonyme Stenocline gymnocephala DC.. Il a pour noms vernaculaires locaux Rambazina, Rambiasina. Cette plante est présente de 800 à 2000 mètres d'altitude, sur toutes les zones déforestées. Elle fait partie de la végétation secondaire qui se développe après abandon de la culture.

Helichrysum gymnocephalum est traditionnellement utilisé en thérapeutique sous forme de tisanes ou en sirop préparé à partir des feuilles pour soigner les aphtes, les gingivites ou les ulcères ou sous forme de pommade par mélange des feuilles broyées avec du saindoux, contre les rhumatismes et la goutte. En décoction, il soulage les céphalées. Les feuilles et les sommités fleuries ont des propriétés diurétiques, fortifiantes et soulageraient les névralgies.
WO 2012/098134 divulgue un extrait de *Helichrysum gymnocephalum* destiné au traitement de l'hyperpigmentation de la peau (voir page 3, lignes 21-22).

Afoulous S. et al.("Helichrysum gymnocephalum essential oil: chemical composition and cytotoxic, antimalarial and antioxidant activities, attribution of the activity origin by corrélations", Molecules: a journal of synthetic organic and natural product chemistry, 2011, 16, p. 8273-8291) divulgue une huile essentielle d*'Helichrysum gymnocephalum* préparée par hydrodistillation de ses feuilles. Cette huile essentielle possède une bonne activité anticancéreuse et antipaludéenne avec un faible pouvoir antioxydant.

L'étude bibliographique (De Medicis et al., 1992 ; Mollenbeck et al., 1997) indique qu'il existe une grande variabilité phénotypique pour les espèces du genre Helichrysum. On rencontre également des problèmes de variabilité intra spécifique et interspécifique. Des études ont notamment été réalisées sur l'huile essentielle d'Helichrysum gymnocephalum. Ces études ne mentionnent aucunement la présence de polyphénols chez cette espèce. En revanche, il est connu que le genre Helichrysum contient des polyphénols acylés de type phloroglucinols (Rosa et al., 2007) et des chalcones (Lall et al., 2006). Une nouvelle chalcone dénommée gymnochalcone a notamment été identifiée dans Helichrysum gymnocephalum, une telle molécule ayant une activité dépigmentante (WO 2012/098134) .

Les dermatoses sont des affections de la peau et des muqueuses, qui se caractérisent par des manifestations inesthétiques comme des rougeurs et des plaques de desquamation. Plusieurs pathologies sont regroupées sous la dénomination de dermatoses inflammatoires. On peut citer, à titre d'exemples non limitatifs, la dermatite atopique, l'eczéma, le psoriasis, la rosacée, le lichen plan, les prurigos, les dermites séborrhéiques ou encore l'acné. Ces dermatoses résultent bien souvent de phénomènes inflammatoires et de désordres immunitaires.

La dermatite atopique est la manifestation cutanée de l'atopie. C'est une dermatose inflammatoire chronique, survenant sur un terrain génétiquement déterminé. Elle touche 15 à 30% des enfants et 2 à 10% des adultes. Sa prévalence est en augmentation constante dans les pays industrialisés ; elle a doublé, voire triplé, au cours des trois dernières décennies et elle est maintenant considérée comme une préoccupation majeure de la santé publique. La dermatite atopique est souvent associée à d'autres désordres atopiques, tels que la rhinite allergique et l'asthme. Cette affection apparaît le plus souvent au cours de la petite enfance et se caractérise par des éruptions répétées pendant plusieurs années. Elle évolue par poussées entrecoupées de rémissions spontanées. Les lésions se caractérisent par une sécheresse cutanée importante et par des lésions inflammatoires : éruptions érythémateuses papuleuses, vésiculeuses, squameuses et très prurigineuses. Sur le plan histologique, la dermatite atopique se caractérise, comme bien des dermatoses, par une infiltration de lymphocytes, monocytes et de polynucléaires éosinophiles autour de petits vaisseaux et des capillaires ; sur le plan biochimique, elle se caractérise par l'expression de cytokines telle que la Thymic Stromal Lymphopoietin (TSLP) ; par ailleurs, il a été montré que les chimiokines, notamment l'interleukine 8 (IL8) et les médiateurs lipidiques de l'inflammation tels que la prostaglandine 6KF1α (PG6KF1α), sont fortement impliquées dans les dermatoses telles que la dermatite atopique et dans les maladies inflammatoires chroniques en général.

L'eczéma est une dermatose prurigineuse caractérisée par une inflammation non contagieuse de la peau qui s'accompagne de rougeurs, de fines vésicules, de squames et de démangeaisons. Il peut commencer très tôt dans la vie, il s'observe même chez le nourrisson. Les personnes atteintes connaissent des périodes communément appelées « poussées d'eczéma », durant lesquelles les symptômes s'aggravent. Ces poussées, de durée variable, sont entrecoupées de périodes de rémission. L'eczéma serait un désordre de nature génétique, mais des facteurs environnementaux tels que la présence d'irritants chimiques ou le stress influenceraient son apparition.

Le psoriasis, maladie inflammatoire de la peau par excellence, se caractérise par l'apparition d'épaisses plaques de peau qui se desquament. Ces plaques sont présentes à différents endroits du corps, le plus souvent sur les coudes, les genoux et le cuir chevelu. Cette maladie chronique évolue par cycles, avec des périodes de rémission. Le psoriasis peut être très désagréable voire même douloureux lorsqu'il se manifeste sur la paume des mains, la plante des pieds ou dans les plis de la peau. Il existe plusieurs types de psoriasis, la forme la plus courante étant le psoriasis en plaques ou psoriasis vulgaire. Les autres formes sont le psoriasis en gouttes, érythrodermique et pustuleux.

La rosacée est une dermatose inflammatoire commune chronique et progressive liée à une relaxation vasculaire. Il s'agit d'une affection qui touche les petits vaisseaux du visage. Elle touche fréquemment les personnes à peau claire et peut avoir des conséquences psycho-affectives importantes. Le nom de cette pathologie fait référence à la couleur caractéristique que prend le visage lors de la maladie.

Le lichen plan est une éruption cutanée prurigineuse ne touchant que les adultes. Il s'agit d'une affection cutanée inflammatoire d'origine inconnue. Cette dermatose touche la peau, les muqueuses mais aussi les cheveux et les ongles. Ces deux dernières localisations sont en général d'évolution chronique et peuvent laisser des séquelles irréversibles, telles que alopécie et destruction des ongles.

Un prurigo est un prurit intense de la peau avec des papules érythémateuses et vésiculeuses avec lésions de grattage. Il s'agit le plus souvent d'une sensibilité exagérée aux piqures d'insectes, sensibilité qui se prolonge anormalement longtemps et qui est fréquente surtout chez le jeune enfant.

La dermite séborrhéique est une affection inflammatoire chronique de la peau, qui atteint les zones riches en glandes sébacées, à savoir le cuir chevelu et le visage. Elle est due à une levure, la Malassezia, présente dans la peau et qui se développe dans le sébum. Cette affection évolue par poussées favorisées par le stress, l'absence de soleil et la pollution.

L'acné est une pathologie cutanée commune, résultat d'une inflammation des follicules pilo-sébacés dus en grande partie à la colonisation de *Propionibacterium acnés* dans l'infundibulum.

Dans le cas d'affections légères d'une dermatose inflammatoire, des émollients et des kératolytiques sont préconisés. Ces traitements ont pour but de rendre les lésions tolérables pour le malade et ils n'ont souvent qu'un effet suspensif. Pour les affections plus sévères, ce sont des antiinflammatoires ou des corticoïdes susceptibles de réguler l'inflammation des kératinocyte, qui sont utilisés depuis plusieurs années. Tous ces traitements ont des effets secondaires importants, parfois très lourds pour les patients. Du fait des effets secondaires importants des traitements existants précités pour les troubles cutanés ou du cuir chevelu résultant de l'état d'activation de la réponse immunitaire innée et inflammatoire épidermique de la peau, il existe un réel besoin de disposer de nouveaux actifs cosmétiques ou thérapeutiques et de nouvelles compositions cosmétiques ou thérapeutiques utilisables pour le traitement desdits troubles cutanés ou du cuir chevelu.

La présente invention a pour objet de répondre à ces besoins. En effet, les inventeurs ont mis en évidence, de façon tout à fait inattendue, qu'un extrait d'Helichrysum gymnocephalum présente des activités pharmacologiques d'intérêt pour le traitement et la prévention des dermatoses inflammatoires, et plus particulièrement de la dermatite atopique.

Un premier objet de l'invention concerne par conséquent un extrait à l'éthanol d'Helichrysum gymnocephalum pour son utilisation dans le traitement et/ou la prévention des dermatoses inflammatoires.

Au sens de la présente invention, le terme « prévention » signifie éviter l'apparition d'une maladie, d'un trouble ou d'un ou plusieurs signes et/ou symptômes.

D'une façon préférée, les dermatoses inflammatoires sont choisies parmi la dermatite atopique, l'eczéma, le psoriasis, la rosacée, le lichen plan, les prurigos, les dermites séborrhéiques et l'acné. De préférence, il s'agit de la dermatite atopique.

Ledit extrait d'Helichrysum gymnocephalum est avantageusement obtenu à partir des parties aériennes de la plante (par ex. tiges et/ou feuilles) ou de la plante entière, fraîche(s) ou sèche(s), entières, coupées ou broyées, soumise(s) à au moins une extraction par de l'éthanol..

L'extraction est avantageusement réalisée sous agitation ou de façon statique, à une température comprise entre la température ambiante et le reflux. Elle peut être assistée par les ultra-sons, par les micro-ondes ou par extrusion. Elle peut être mise en oeuvre dans un ratio poids de plante / volume de solvant pouvant varier de 1/3 à 1/30. L'étape d'extraction est généralement effectuée pendant une durée de 1 minute à 48H. L'extraction peut être renouvelée 2 ou 3 fois.

Le marc est ensuite séparé de l'extrait par centrifugation ou filtration.

L'extrait obtenu peut être décoloré si nécessaire, par exemple sur charbon actif, de façon à éliminer tout ou partie des chlorophylles. Il peut ensuite être plus ou moins concentré, de manière à obtenir un extrait concentré ou un extrait sec.

Selon un mode de réalisation particulier de l'invention, l'extrait d'Helichrysum gymnocephalum selon l'invention est caractérisé par une teneur de 2 à 40%, notamment 5 à 25%, des trois flavonoïdes galangine, pinobanksine et cardamonine (% en poids / poids sec de l'extrait).

Selon un mode de réalisation particulier de l'invention, l'extrait d'Helichrysum gymnocephalum selon l'invention est obtenu à partir des parties aériennes sèches de la plante.

Un autre objet de l'invention concerne un prémélange, comprenant un extrait à l'éthanol d'Helichrysum gymnocephalum selon l'invention et au moins un support, pour son utilisation dans le traitement et/ou la prévention des dermatoses inflammatoires.

En effet, dans un mode de réalisation particulier de l'invention, un support peut être ajouté à l'extrait selon l'invention avant l'étape de concentration de façon à obtenir un prémélange selon l'invention après évaporation totale ou partielle, de préférence totale, du solvant d'extraction.

Le support peut être tout support cosmétologiquement acceptable. Il peut s'agir en particulier de la maltodextrine, du lactose, de la silice, du glycérol, ou d'un mélange de ceux-ci. Selon un mode de réalisation particulier, il s'agira de maltodextrine.

Le prémélange pourra contenir 20 à 95 % en poids de support par rapport au poids total du prémélange sec.

Le prémélange pourra contenir 5 à 80 % en poids d'extrait sec selon l'invention par rapport au poids total du prémélange sec.

Un autre objet de la demande de brevet concerne une composition dermatologique ou dermo-cosmétique comprenant à titre de principe actif au moins un extrait à l'éthanol d'Helichrysum gymnocephalum ou un prémélange selon l'invention avec au moins un excipient dermatologiquement ou dermo-cosmétiquement acceptable, pour son utilisation dans le traitement et/ou la prévention des dermatoses inflammatoires.

Dans un mode de réalisation particulier, les dermatoses inflammatoires sont choisies parmi la dermatite atopique, l'eczéma, le psoriasis, la rosacée, le lichen plan, les prurigos, les dermites séborrhéiques et l'acné.

Selon un autre mode de réalisation particulier, la composition selon l'invention est utilisée dans le traitement et/ou la prévention du prurit.

De façon préférée, la composition selon l'invention est utilisée dans le traitement et/ou la prévention de la dermatite atopique.

Dans un mode de réalisation particulier, la composition dermatologique ou dermo-cosmétique selon l'invention comprend 0,001% à 5%, préférentiellement 0,01% à 1% en poids d'extrait sec d'un extrait d'Helichrysum gymnocephalum selon l'invention, par rapport au poids total de la composition.

Dans la présente invention, on entend désigner par « dermatologiquement ou dermo-cosmétiquement acceptable » ce qui est utile dans la préparation d'une composition dermatologique ou dermo-cosmétique, qui est généralement sûr, non toxique et ni biologiquement ni autrement non souhaitable et qui est acceptable pour une utilisation dermatologique ou dermo-cosmétique notamment par application topique.

Les compositions selon l'invention sont avantageusement destinées à une application topique, notamment par application sur la peau.

Par « application topique », on entend une application sur la peau, les muqueuses et/ou les phanères.

Les compositions selon l'invention pourront ainsi se présenter sous les formes qui sont habituellement connues pour une administration topique, c'est-à-dire notamment les lotions, les mousses, les gels, les dispersions, les émulsions, les sprays, les sérums, les baumes, les masques ou les crèmes, avec des excipients permettant notamment une pénétration cutanée afin d'améliorer les propriétés et l'accessibilité du principe actif. Avantageusement il s'agira d'une crème, ou d'une émulsion.

L'invention vise ainsi des compositions dermatologiques ou dermo-cosmétiques selon l'un des modes de réalisation de la présente invention, caractérisées ne ce qu'elles se présentent sous une forme propre et adaptée à une application topique.

Ces compositions contiennent généralement, outre l'extrait selon la présente invention, un milieu physiologiquement acceptable, en général à base d'eau ou de solvant, par exemple des alcools, des éthers ou des glycols. Elles peuvent également contenir des agents tensioactifs, des agents complexant, des conservateurs, des agents stabilisants, des émulsifiants, des épaississants, des gélifiants, des humectants, des émollients, des oligo-éléments, des huiles essentielles, des parfums, des colorants, des agents matifiants, des filtres chimiques ou minéraux, des agents hydratants ou des eaux thermales, etc.

Les exemples qui suivent sont destinés à illustrer l'invention sans en limiter la portée.

### FIGURES

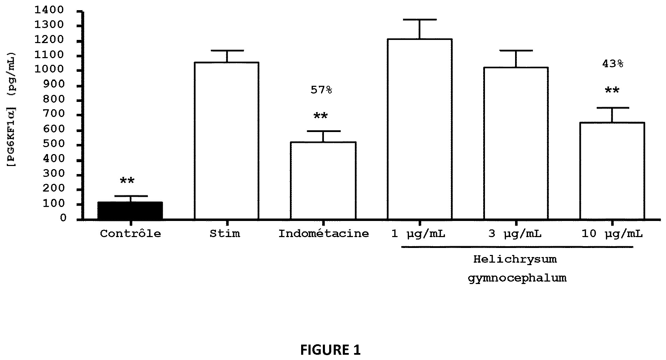

La **Figure 1** représente la production de prostaglandine PG6KF1α (en pg/mL) d'une culture de kératinocytes contrôle, après stimulation à l'ionophore calcique A23187 (Stim), après ajout d'indométacine, ou après ajout d'un extrait d'Helichrysum gymnocephalum selon l'invention à 1, 3 ou 10 µg/mL.

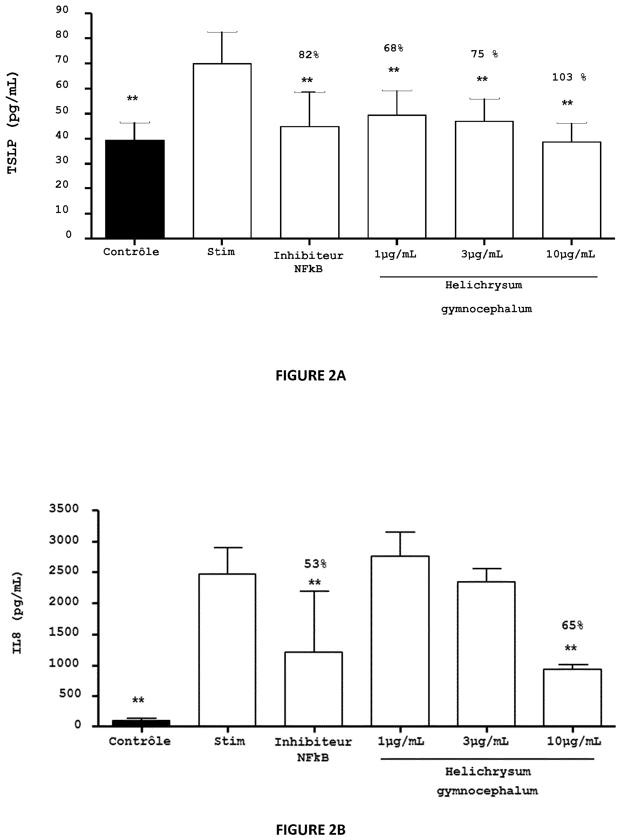

La **Figure 2** représente la production (**A**) de TSLP (en pg/mL) et (B) de IL8 (en pg/mL) d'une culture de kératinocytes contrôle, après stimulation (Stim), après ajout d'un inhibiteur NFkB, ou après ajout d'un extrait d'Helichrysum gymnocephalum selon l'invention à 1, 3 ou 10 pg/mL.

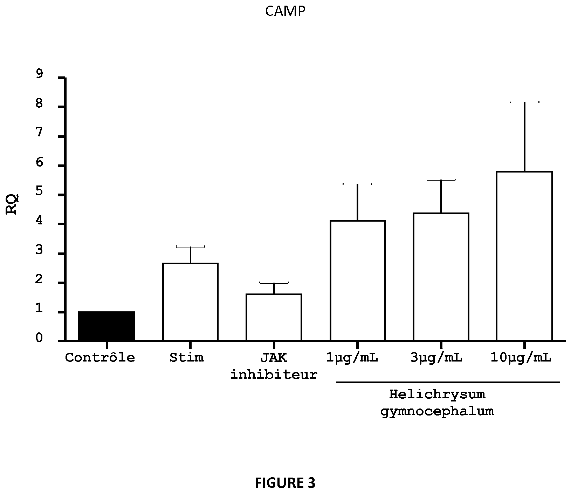

La **Figure 3** représente l'expression de CAMP (Cathelicidin Antimicrobial Peptide) (quantification relative - RQ) dans une culture de kératinocytes contrôle, après stimulation (Stim), après ajout d'un inhibiteur JAK, ou après ajout d'un extrait d'Helichrysum gymnocephalum selon l'invention à 1, 3 ou 10 pg/mL.

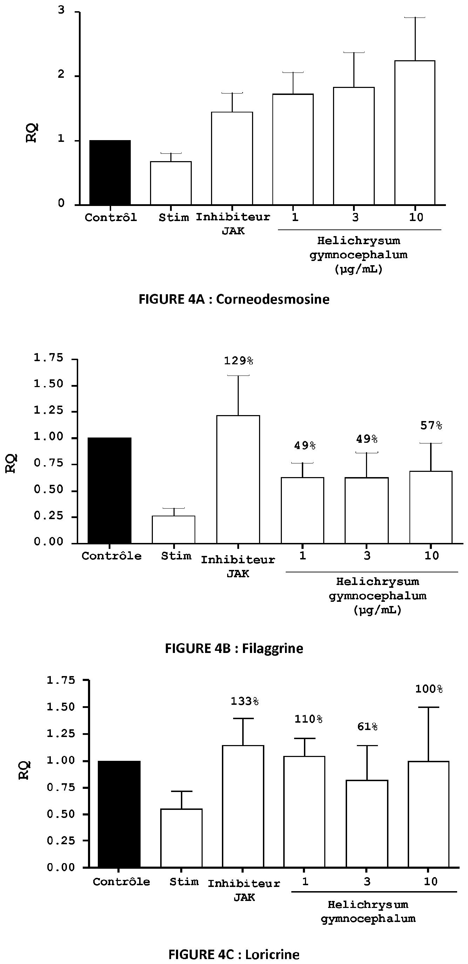

La **Figure 4** représente l'expression (**A**) de la cornéodesmosine, (**B**) de la filaggrine et (**C**) de la loricrine (quantification relative - RQ) dans une culture de kératinocytes contrôle, après stimulation (Stim), après ajout d'un inhibiteur JAK, ou après ajout d'un extrait d'Helichrysum gymnocephalum selon l'invention à 1, 3 ou 10 pg/mL.

### REFERENCES

De Medici D., Pieretti S., Salvatore G., Nicoletti M., Rasoanaivo P. Chemical analysis of essential oils of malagasy médicinal plants by gas chromatography and NMR spectroscopy. Flavour Fragr J. 7, 275-281, 1992.

Lall N., Hussein A. A., Meyer J. J. M. Antiviral and antituberculous activity of Helichrysum melanacme constituents. Fitoterapia. 77, 230-232, 2006.

Möllenbeck S., König T., Schreier P., Schwab W., Rajaonarivony L. Chemical composition and analyses of enantiomers of essential oils from Madagascar. Flavour Fragr J. 12, 63-69, 1997.

Rosa A., Deiana M. Atzeri A., Giulia C., Incani A. Evaluation of the antioxidant and cytotoxic activity of arzanol, a prenylated α-pyrone-phloroglucinol heterodimer from Helichrysum italicum subsp. Microphyllum. Chemico-Biological Interactions. 165, 117-126, 2007.

### WO 2012/098134

### EXEMPLES

### I - Préparation d'un extrait ou prémélange contenant un extrait utilisé pour l'invention

### Exemple 1 : extraction à reflux à l'éthanol 96

2,5kg de parties aériennes sèches broyées d'Helichrysum gymnocephalum sont extraites à reflux sous agitation par 50 litres d'éthanol 96% (v/v) pendant 1 heure dans un réacteur. L'extrait est ensuite filtré sur filtre K900 et le solvant est évaporé de manière à obtenir 400g d'une pâte verte avec un rendement de 16% et contenant 11,2% des 3 flavonoïdes (galangine, pinobanksine, cardamonine).

### Exemple 2 : extraction à température ambiante à l'éthanol 96

100g de parties aériennes sèches d'Helichrysum gymnocephalum non broyées sont extraites à température ambiante sous agitation par 2 litres d'éthanol 96% (v/v) pendant 1 heure dans un réacteur. L'extrait est ensuite filtré sur filtre K900 et le solvant est évaporé de manière à obtenir 10g d'une pâte marron-orange avec un rendement de 10% et contenant 12,2% des 3 flavonoïdes.

### Exemple 3 : extraction à température ambiante à l'éthanol 90 et mise sur support maltodextrine

250g de parties aériennes sèches d'Helichrysum gymnocephalum sont extraites par 5 litres d'éthanol 90% (v/v). Après filtration sur K900, l'extrait est concentré puis séché sur maltodextrine de façon à obtenir 62g de prémélange sous forme de poudre vert-marron, avec un rendement de 25%, contenant 70% de maltodextrine et 5% des 3 flavonoïdes.

### Exemple 4 : extraction à température ambiante à l'éthanol 90 suivi d'une décoloration au charbon actif et mise sur support maltodextrine

250g de parties aériennes sèches d'Helichrysum gymnocephalum sont extraites par 5 litres d'éthanol 90% (v/v). Après filtration sur K900, l'extrait est concentré puis décoloré au charbon actif (30% p/p par rapport à la matière sèche). Après filtration il est séché sur maltodextrine de façon à obtenir 61g de prémélange sous forme de poudre marron-orange, avec un rendement de 24% contenant 70% de maltodextrine et 4,4% des 3 flavonoïdes.

### Exemple 5 : extraction à reflux à l'acétate d'éthyle (hors invention)

50 g de parties aériennes sèches et broyées d'Helichrysum gymnocephalum sont extraites par 500 mL d'acétate d'éthyle à reflux pendant 1H. Après filtration, l'extrait est séché de façon à obtenir 6,25g de pâte verte (rendement 12,5%) contenant 5,6% des 3 flavonoïdes.

### Exemple 6 : extraction au CO₂ supercritique (hors invention)

200 g de parties aériennes séchées et broyées d'Helichrysum gymnocephalum sont extraites à 200 bars et 57°C par du CO₂ supercritique (débit 10 kg/H) pendant 240 minutes. L'extrait est ensuite récupéré sous forme d'une cire jaune avec un rendement de 9% et une teneur des 3 flavonoïdes de 8,1%.

### Exemple 7 : extraction à l'éthanol 90 assistée par ultra-sons

15g de plante entière séchée et broyée sont mis en contact avec 300 mL d'éthanol 96° puis extraits sous l'action des ultra-sons (20 kHz) pendant 1 minute à une amplitude de 100%. Après filtration, le solvant est évaporé conduisant à l'obtention de 1,7 g de pâte marron-orange (rendement 11%) contenant 23,7% des 3 flavonoïdes.

### II - Evaluation pharmacologique

### Exemple 8 : Activité anti-inflammatoire d'un extrait d'Helichrysum gymnocephalum

Le kératinocyte est la cellule la plus représentée dans l'épiderme. En réponse à plusieurs facteurs extracellulaires présents dans l'environnement, l'épiderme libère divers médiateurs biologiquement actifs, en particulier des lipides bioactifs tels que des prostaglandines et des leucotriènes, qui jouent un rôle important dans l'initiation et la modulation des réactions inflammatoires de la peau mais qui participent aussi dans la régulation de la réponse immune.

Le kératinocyte apparaît comme un bon modèle d'étude pharmacologique de la peau. Ce modèle cellulaire permet de déterminer *in vitro* les capacités de divers composés à moduler la production de ces médiateurs résultant du métabolisme de l'acide arachidonique.

Dans cette étude, les inventeurs ont investigué une prostaglandine, la 6-kéto-PGF1α (PG6KF1α), qui est le métabolite stable de la prostacycline. Cette dernière est l'un des métabolites majeurs produits par les kératinocytes stimulés, et est représentatif de la modulation de production des métabolites de l'acide arachidonique (Dorris et Stokes Peebles, Mediators of Inflammation, vol 2012, article ID 926968, 9 pages, 2012).

Le but de cette étude est de rechercher une activité anti-inflammatoire potentielle d'un extrait d'Helichrysum gymnocephalum préparé selon l'exemple 2, en mesurant ses effets sur la libération de prostaglandine PG6KF1α.

La lignée cellulaire utilisée dans cette étude est la lignée HaCat (kératinocytes humains).

Les cellules sont mises en culture dans du milieu DMEM (Dulbecco's Modified Eagle Medium) complémenté par du sérum de veau foetal dans un environnement à 37°C et 5% CO₂ sur plaques de 24 puits. Elles sont ensuite pré-incubées pendant 60 minutes avec les produits à tester. Un agent stimulant de la voie de l'acide arachidonique est ajouté pendant 5 heures, à savoir l'ionophore calcique A23187 (solution dans du DMSO à 0,01%) utilisé à 5µM. Après 5 heures de stimulation, le surnageant de culture de chaque puit est prélevé, centrifugé à 3000 tours/min puis stocké à -20°C. La production de prostaglandine PG6KF1α dans le surnageant de culture est mesurée avec le kit Euromedex Elisa selon les instructions du fournisseur. L'analyse statistique est réalisée par une ANOVA suivi du post test de Dunnett. Il est à noter que 3 expériences indépendantes ont été menées. Les résultats pour chaque produit et pour chaque dose sont moyennés à partir des mesures faites sur 3 puits.

Les résultats sont représentés dans la figure 1. La stimulation (mentionné stim sur la figure 1) par l'ionophore calcique A23187 stimule fortement la production de PG6KF1α, l'indométacine (anti-inflammatoire non stéroïdien) inhibe cette production de plus de 50%, ce qui permet de valider la pertinence de ce test. L'extrait d'Helichrysum gymnocephalum (utilisé sous forme de solution dans le DMSO de concentration 100 mg/mL) inhibe également la production de PG6KF1α dès 10µg/mL, avec plus de 40% d'inhibition (p<0,01 versus la condition stimulée). Les inventeurs ont donc mis en évidence que l'extrait d'Helichrysum gymnocephalum possède une activité anti-inflammatoire et donc présente des propriétés apaisantes.

### Exemple 9 : Activité d'un extrait d'Helichrysum gymnocephalum sur un modèle de dermatite atopique, activité sur TSLP et IL8

La dermatite atopique serait la résultante d'interactions complexes entre les prédispositions génétiques, le dysfonctionnement de la barrière cutanée, la dysrégulation du système immunitaire et les facteurs environnementaux, notamment les allergènes et microorganismes. A ce jour, les biomarqueurs spécifiquement associés à la sévérité de la dermatite atopique incluent des marqueurs protéiques tels que la cytokine TSLP, la chimiokine TARC/CCL17, l'interleukine 4, le taux élevé d'immunoglobuline E et la protéine de structure filaggrine qui est sous-exprimée.

Le système immunitaire cutané est capable de développer deux types de réponse : inné et adaptatif. L'immunité innée est la première défense de l'organisme contre les infections cutanées, elle permet une réponse immédiate aux pathogènes. Les kératinocytes jouent un rôle majeur dans la réponse immune, ils ont notamment la capacité de reconnaître les pathogènes, de produire des molécules antimicrobiennes et d'émettre des signaux pro-inflammatoires.

La dermatite atopique est également caractérisée par une inflammation de la peau. L'inflammation cutanée est un phénomène complexe qui met en jeu de nombreuses cascades de signalisations.

Le prurit est l'un des symptômes de la dermatite atopique, il est défini comme une sensation déplaisante qui provoque le besoin de se gratter. Les médiateurs impliqués dans le prurit sont nombreux, mais trois voies prédominent : la voie histaminergique notamment avec les récepteurs H1 et H4, la voie avec la cytokine TSLP et une voie non-histaminergique dépendante de PAR-2, ce dernier appartenant également à la famille des récepteurs couplés aux protéines G. Ces récepteurs sont activés par clivage protéolytique et permettent d'activer diverses voies de signalisation intracellulaires, ils régulent la différentiation des kératinocytes, participent à l'initiation de l'inflammation. Le prurit de la dermatite atopique provoque de fortes démangeaisons chez le patient, il génère des lésions cutanées qui sont le siège de la production et de la libération par les kératinocytes de cytokines pro-inflammatoires qui ellesmêmes induisent la production de chimiokines et de molécules d'adhésions favorisant le recrutement, la prolifération et la survie des leucocytes. Ces leucocytes infiltrés au niveau cutané entretiennent l'inflammation. Parallèlement, du fait d'une fonction barrière altérée, la pénétration accrue d'antigènes et d'allergènes de l'environnement favorise l'activation des cellules dendritiques résidentes qui, en phase aigüe de la dermatite atopique, induisent alors une polarisation Th2 des lymphocytes T naïfs. Cette polarisation est en partie dépendante de la cytokine TSLP libérée par les kératinocytes chez les patients atteints de dermatite atopique. Les cellules Th2 produisent les interleukines 4 et 13, capables de limiter la production de peptides antimicrobiens, cette diminution entraine une augmentation de la susceptibilité de colonisation par des agents pathogènes tels que S. aureus. En effet, la peau de 90% des patients atteints de dermatite atopique est colonisée par ce pathogène. Des molécules pro-inflammatoires sont alors relarguées et sont responsables d'une exacerbation de l'activation des leucocytes et du cycle de l'inflammation de la peau. De plus, la libération de nombreux médiateurs va perpétuer la sensation du prurit, responsable du cercle vicieux et continu : prurit - démangeaisons - lésions - inflammation.

Dans cette étude, les inventeurs ont évalué les effets d'Helichrysum gymnocephalum préparé selon l'exemple 2 sur la production de cytokines induite par deux alternatives de mélanges inflammatoires :
- Poly (I:C) + IL1α
- IL4 + IL13 + Poly (I:C) + PamC3 sur des kératinocytes humains normaux de l'épiderme.

La stimulation avec Poly (I:C) est réalisée pour induire un phénotype mimant la phase d'initiation de la dermatite atopique, notamment à travers l'induction de TSLP. Ce dernier joue un rôle clé dans les pathologies liées aux allergies, telle que la dermatite atopique La seconde stimulation mime plutôt les deux étapes de la dermatite atopique, la phase d'initiation et la phase chronique.

L'étude a été menée sur des kératinocytes humains de l'épiderme, dans des conditions de culture classiques, à savoir dans un milieu de culture keratinocyte-SFM supplémenté avec EGF (Epidermal Growth Factor) à 0,25 ng/ml et gentamycine 25 µg/ml et un environnement à 37°C et avec 5% de CO₂.

### Modèle de stimulation avec Poly(I:C) et Ilα

Les kératinocytes étaient pré-incubés pendant 24 heures dans un milieu d'essai contenant ou pas (contrôle, DMSO à 0.02%) le composé à tester, ou les composés de référence (un inhibiteur de l'activation de NFκB à 5µM ou la bafilomycine à 100 nM, un inhibiteur de la pompe à protons). Les cellules sont ensuite stimulées avec Poly(I:C) et Ilα. Une condition contrôle non-stimulée est également réalisée en parallèle. Puis les cellules sont incubées pendant 4 heures. Trois expérimentations indépendantes ont été réalisées.

### Modèle de stimulation avec IL4, IL13,Poly(I:C) et PamC3

Les kératinocytes étaient pré-incubés pendant 1 heure dans un milieu d'essai contenant ou pas (contrôle, DMSO à 0.02%) le composé à tester, ou un composé de référence (desonide à 100 µM, corticostéroïde, inhibiteur classique de l'inflammation). Après la pré-incubation, la stimulation est réalisée avec l'ajout des ligands TLR (Poly(I:C) et PamC3 et le mélange de cytokines inflammatoires IL4 et IL13) aux cellules. Une condition contrôle non-stimulée est également réalisée en parallèle. Les cellules sont ensuite incubées pendant 24 heures.

Les surnageants de culture sont récoltés, centrifugés et congelés à -80°C. TSLP et IL8 sont quantifiés par ELISA.

Les résultats sont exprimés en pourcentage d'inhibition de production de TSLP ou d'IL8 par les kératinocytes stimulés.

La signification statistique (p<0.05 ou p<0.01) est déterminée en utilisant une ANOVA à une voie suivie d'un test de Dunnett. Les concentrations de TSLP et IL8 sont moyennées à partir des trois expérimentations indépendantes.

Les résultats sont résumés sur la Figure 2. Les stimulations (mentionnées stim sur la Figure 2) pour mimer l'initiation et la phase chronique de la dermatite atopique permettent d'induire la production de TSLP (Figure 2A) et d'IL8 (Figure 2B) par les kératinocytes humains normaux de l'épiderme. L'inhibiteur de NFκB inhibe significativement la production de ces deux marqueurs, ce qui permet de valider la pertinence de ces tests. L'extrait d'Helichrysum gymnocephalum inhibe de façon concentration-dépendante la production de TSLP allant même jusqu'à 100% d'inhibition à 10pg/mL. De même l'extrait d'Helichrysum gymnocephalum inhibe de façon concentration-dépendante la production d'IL8 avec 65% d'inhibition à la concentration de 10µg/mL.

Les inventeurs démontrent ainsi, dans un environnement de dermatite atopique que l'extrait d'Helichrysum gymnocephalum possède des propriétés apaisantes en inhibant des cytokines inflammatoires sécrétées par les kératinocytes et inducteur de prurit.

### Exemple 10 : Activité d'un extrait d'Helichrysum gymnocephalum sur l'induction de gène de la réponse immunitaire innée dans un modèle de dermatite atopique

Les peptides antimicrobiens apparentés à la cathélicidine sont une famille de polypeptides que l'on trouve dans les lysosomes des macrophages, les leucocytes polymorphonucléaires et les kératinocytes. Les cathélicidines jouent un rôle essentiel dans la défense immunitaire innée contre les infections bactériennes invasives. La CAMP (Cathelicidin antimicrobial peptide, LL37) est un peptide antimicrobien produit par les kératinocytes et sous exprimé chez les individus atteints de dermatite atopique.

Dans cette étude, les inventeurs ont évalué les effets d'Helichrysum gymnocephalum préparé selon l'exemple 2 sur l'induction de CAMP induit par un mélange inflammatoire Poly(I:C) et IL1α.

Le modèle de stimulation est le même que celui présenté dans l'exemple 9. Les conditions expérimentales sont inchangées.

Les résultats de cette expérience sont résumés sur la Figure 3. La stimulation des kératinocytes par un environnement de dermatite atopique induit une augmentation de l'expression de CAMP. En présence d'un inhibiteur de JAK, l'augmentation de cette expression de CAMP est fortement réduite, ce qui permet de bien valider la pertinence de ce test. L'extrait d'Helichrysum gymnocephalum induit une augmentation de l'expression de CAMP dès 10pg/mL avec une tendance à partir de 1µg/mL.

Les inventeurs démontrent bien que l'extrait d'Helichrysum gymnocephalum induit les défenses immunitaires innées dans un modèle de dermatite atopique.

### Exemple 11 : Activité d'un extrait d'Helichrysum gymnocephalum sur l'induction de l'expression de gènes de la différenciation des kératinocytes dans un modèle de dermatite atopique

L'épiderme joue un rôle protecteur majeur en procurant une barrière chimique et mécanique pour le corps. Elle assure le maintien de l'étanchéité, à savoir la fonction barrière cutanée. Les cornéocytes, kératinocytes de la couche cornée, associés à une matrice lipidique assurent en grande partie cette fonction. Néanmoins les couches plus profondes interviennent dans la mise en place des acteurs de cette fonction. La capacité de différenciation des kératinocytes de l'épiderme va garantir la mise en place d'une barrière de type perméabilité sélective fonctionnelle. D'un point de vue protéique, la différenciation épidermique est majoritairement axée sur l'évolution de protéines de structure que sont les kératines et qui contribuent à l'intégrité architecturale de l'épiderme. Leur expression varie en fonction du degré de maturation des kératinocytes. Ainsi, il existe des marqueurs précoces de la différenciation des kératinocytes comme la kératine 1 basique, et des marqueurs plus tardifs comme la cornéodesmosine, protéine de l'enveloppe cornée. Ainsi, la cornéodesmosine assure la cohésion de la barrière cutanée. La formation de la matrice fibreuse présente dans les cornéocytes est initiée au niveau de la transition entre kératinocytes granuleux et les cornéocytes. Les molécules basiques de filaggrine produites à partir de son précurseur la profilaggrine s'associent aux filaments de cytokératine, permettant ainsi leur aggrégation. La filaggrine est un marqueur de la différenciation des kératinocytes. La loricrine est une protéine qui s'observe sous forme de granules dans le cytoplasme des kératinocytes du stratum granulosum. Les grains de loricrine, très basophiles sont détectables en immunohistochimie. La loricrine n'est associée à aucune structure morphologiquement individualisable. La loricrine est également un marqueur de la différenciation des kératinocytes.

Les inventeurs ont évalué les effets potentiels d'un extrait d'Helichrysum gymnocephalum préparé selon l'exemple 2 sur la régulation de cibles moléculaires impliquées dans les processus de différenciation kératinocytaire. Pour cela, les conditions expérimentales sur les kératinocytes normaux humains sont les mêmes que celles décrites dans l'exemple 9. L'environnement de la dermatite atopique est mimé par la stimulation des kératinocytes avec Poly(I:C) et IL1α.

Les résultats sont représentés sur la figure 4. La stimulation des kératinocytes avec le cocktail (poly(I:C) et IL1α) induit une moindre expression des gènes étudiés, que dans les conditions contrôles. En effet ce protocole mime une dematite atopique, dans ces conditions la barrière cutanée est altérée et de ce fait les gènes impliqués dans la différenciation des kératinocytes ont leur expression qui diminue. L'ajout d'un inhibiteur JAK induit une augmentation de l'expression des 3 gènes étudiés. Ces deux résultats permettent de valider la pertinence de ce modèle. L'extrait d'Helichrysum gumnocephalum induit une augmentation de l'expression de la cornéodesmosine (Figure 4A), de la filaggrine (Figure 4B) et de la loricrine (Figure 4C) dès 1 µg/mL dans un environnement de dermatite atopique.

Les inventeurs démontrent que l'extrait d'Helichrysum gymnocephalum favorise la restauration de la barrière cutanée dans un environnement de dermatite atopique.

## Revendications

1. Extrait à l'éthanol d'Helichrysum gymnocephalum pour son utilisation dans le traitement et/ou la prévention des dermatoses inflammatoires.

2. Extrait à l'éthanol d'Helichrysum gymnocephalum pour son utilisation selon la revendication 1, **caractérisé en ce que** l'extrait à l'éthanol d'Helichrysum gymnocephalum est obtenu à partir des parties aériennes sèches de la plante.

3. Extrait à l'éthanol d'Helichrysum gymnocephalum pour son utilisation selon l'une quelconque des revendications 1 et 2, **caractérisé en ce que** les dermatoses inflammatoires sont choisies parmi la dermatite atopique, l'eczéma, le psoriasis, la rosacée, le lichen plan, les prurigos, les dermites séborrhéiques et l'acné.

4. Prémélange comprenant un extrait à l'éthanol d'Helichrysum gymnocephalum selon l'une quelconque des revendications 1 et 2 et un support, pour son utilisation dans le traitement et/ou la prévention des dermatoses inflammatoires.

5. Prémélange pour son utilisation selon la revendication 4, **caractérisé en ce que** le support est choisi parmi la maltodextrine, le lactose, la silice, le glycérol, et un mélange de ceux-ci.

6. Composition dermatologique ou dermo-cosmétique comprenant à titre de principe actif au moins un extrait à l'éthanol d'Helichrysum gymnocephalum ou un prémélange tel que défini à la revendication 4 ou 5 avec au moins un excipient dermatologiquement ou cosmétiquement acceptable, pour son utilisation dans le traitement et/ou la prévention des dermatoses inflammatoires.

7. Composition dermatologique ou dermo-cosmétique pour son utilisation selon la revendication 6, dans laquelle les dermatoses inflammatoires sont choisies parmi la dermatite atopique, l'eczéma, le psoriasis, la rosacée, le lichen plan, les prurigos, les dermites séborrhéiques et l'acné.

8. Composition dermatologique ou dermo-cosmétique selon la revendication 6, pour son utilisation dans le traitement et/ou la prévention de la dermatite atopique.

9. Composition dermatologique ou dermo-cosmétique selon la revendication 6, pour son utilisation dans le traitement et/ou la prévention du prurit.

10. Composition dermatologique ou dermo-cosmétique pour son utilisation selon l'une quelconque des revendications 6 à 9, **caractérisée en ce que** la composition comprend 0,01% à 1% en poids d'extrait sec d'un extrait à l'éthanol d'Helichrysum gymnocephalum par rapport au poids total de la composition.

11. Composition dermatologique ou dermo-cosmétique pour son utilisation selon l'une quelconque des revendications 6 à 10, **caractérisée en ce que** la composition se présente sous une forme propre à une application topique.

## Patentansprüche

1. Ethanolextrakt aus Helichrysum gymnocephalum zur Verwendung bei der Behandlung und/oder Prävention von entzündlichen Dermatosen.

2. Ethanolextrakt aus Helichrysum gymnocephalum zur Verwendung nach Anspruch 1, **dadurch gekennzeichnet, dass** der Ethanolextrakt aus Helichrysum gymnocephalum aus trockenen luftgetragenen Teilen der Pflanze gewonnen wird.

3. Ethanolextrakt aus Helichrysum gymnocephalum zur Verwendung nach einem der Ansprüche 1 und 2, **dadurch gekennzeichnet, dass** entzündliche Dermatosen unter atopischer Dermatitis, Ekzemen, Psoriasis, Rosacea, Lichen planus, Prurigos, seborrhoischer Dermatitis und Akne ausgewählt sind.

4. Vormischung mit einem Ethanolextrakt aus Helichrysum gymnocephalum nach einem der Ansprüche 1 und 2 und einem Träger, zur Verwendung bei der Behandlung und/oder Prävention von entzündlichen Dermatosen.

5. Vormischung zur Verwendung nach Anspruch 4, **dadurch gekennzeichnet, dass** der Träger aus Maltodextrin, Lactose, Kieselsäure, Glycerin und einer Mischung davon ausgewählt ist.

6. Dermatologische oder dermokosmetische Zusammensetzung, die als Wirkstoff wenigstens einen Ethanolextrakt von Helichrysum gymnocephalum oder eine Vormischung gemäß Anspruch 4 oder 5 mit wenigstens einem dermatologisch oder kosmetisch akzeptablen Hilfsstoff zur Verwendung bei der Behandlung und/oder Vorbeugung von entzündlichen Dermatosen umfasst.

7. Dermatologische oder dermokosmetische Zusammensetzung zur Verwendung nach Anspruch 6, wobei die entzündlichen Dermatosen unter atopischer Dermatitis, Ekzemen, Psoriasis, Rosacea, Lichen planus, Prurigos, seborrhoischer Dermatitis und Akne ausgewählt sind.

8. Dermatologische oder dermokosmetische Zusammensetzung nach Anspruch 6, zur Verwendung bei der Behandlung und/oder Prävention von atopischer Dermatitis.

9. Dermatologische oder dermokosmetische Zusammensetzung nach Anspruch 6, zur Verwendung bei der Behandlung und/oder Prävention von Juckreiz.

10. Dermatologische oder dermokosmetische Zusammensetzung zur Verwendung nach einem der Ansprüche 6 bis 9, **dadurch gekennzeichnet, dass** die Zusammensetzung 0,01 Gew.-% bis 1 Gew.-% Trockenextrakt eines Ethanolextrakts von Helichrysum gymnocephalum im Verhältnis zum Gesamtgewicht der Zusammensetzung umfasst.

11. Dermatologische oder dermokosmetische Zusammensetzung zur Verwendung nach einem der Ansprüche 6 bis 10, **dadurch gekennzeichnet, dass** die Zusammensetzung in einer für eine topische Anwendung bestimmten Form vorliegt.

## Claims

1. An ethanol extract of Helichrysum gymnocephalum for use in the treatment and/or prevention of inflammatory dermatoses.

2. The ethanol extract of Helichrysum gymnocephalum for use according to claim 1, **characterised in that** the ethanol extract of Helichrysum gymnocephalum is obtained from the dry aerial parts of the plant.

3. The ethanol extract of Helichrysum gymnocephalum for use according to any of claims 1 and 2, **characterised in that** the inflammatory dermatoses are selected from atopic dermatitis, eczema, psoriasis, rosacea, lichen planus, prurigos, seborrheic dermatitis and acne.

4. A premixture comprising an ethanol extract of Helichrysum gymnocephalum according to any of claims 1 and 2 and a carrier, for use in the treatment and/or prevention of inflammatory dermatoses.

5. The premixture for use according to claim 4, **characterised in that** the carrier is selected from maltodextrin, lactose, silica, glycerol, and a mixture thereof.

6. A dermatological or dermo-cosmetic composition comprising as an active ingredient at least one ethanol extract of Helichrysum gymnocephalum or a premixture as defined in claim 4 or 5 with at least one dermatologically or cosmetically acceptable excipient, for use in the treatment and/or prevention of inflammatory dermatoses.

7. The dermatological or dermo-cosmetic composition for use according to claim 6, wherein the inflammatory dermatoses are selected from atopic dermatitis, eczema, psoriasis, rosacea, lichen planus, prurigos, seborrheic dermatitis, and acne.

8. The dermatological or dermo-cosmetic composition according to claim 6, for use in the treatment and/or prevention of atopic dermatitis.

9. The dermatological or dermo-cosmetic composition according to claim 6, for use in the treatment and/or prevention of pruritus.

10. The dermatological or dermo-cosmetic composition for use according to any of claims 6 to 9, **characterised in that** the composition comprises 0.01% to 1% by weight of dry extract of an ethanol extract of Helichrysum gymnocephalum relative to the total weight of the composition.

11. The dermatological or dermo-cosmetic composition for use according to any of claims 6 to 10, **characterised in that** the composition is in a form specific to a topical application.
